# FASCICULE DE BREVET EUROPEEN

(11) **EP 1 473 025 B1**
(45) Date de publication et mention de la délivrance du brevet: **28.03.2012**
(21) Numéro de dépôt: 04291095.0
(22) Date de dépôt: 28.04.2004
(51) Int. Cl.: A61K 8/87, A61K 8/73, A61Q 5/10, A61K 8/86, A61K 8/41

(54) **Composition tinctoriale comprenant le 2-chloro 6-méthyl 3-amino phénol et un polymère associatif**
Färbemittel enthaltend 2-Chlor-6-Methyl-3-Aminophenol und ein assoziatives Polymer
Dyeing composition containing 2-chloro-6-methyl-3-aminophenol and an associative polymer

(30) Priorité: 29.04.2003 FR 0305242
(43) Date de publication de la demande: 03.11.2004
(73) Titulaire: L'Oréal, 75008 Paris (FR)
(72) Inventeur: Audousset, Marie-Pascale, 92600 Asnieres (FR)
(74) Mandataire: Fevrier, Murielle Françoise E.

(56) Documents cités:
- WO-A-01/41717
- WO-A-02/22095
- WO-A-98/03150
- WO-A-03/017956
- DE-A- 3 016 008
- DE-U- 20 118 982
- FR-A- 2 802 089
- US-A- 6 156 076
- US-A1- 2001 023 514

## Description

L'invention a pour objet une composition pour la teinture d'oxydation des fibres kératiniques, et en particulier des fibres kératiniques humaines telles que les cheveux, comprenant le 2-chloro 6-méthyl 3-amino phénol à titre de coupleur, au moins une base d'oxydation et au moins un polymère associatif épaississant.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

Ces bases d'oxydation et ces coupleurs sont formulés dans des supports permettant leur application sur les fibres kératiniques après mélange avec un agent oxydant.

Pour localiser le produit de coloration d'oxydation à l'application sur les cheveux afin qu'il ne coule pas sur le visage ou en dehors des zones que l'on se propose de teindre, on peut introduire dans ces supports des épaississants traditionnels tels que l'acide polyacrylique réticulé, les hydroxyéthylcelluloses, les cires et des mélanges d'agents tensio-actifs non ioniques de HLB (Hydrophilic Lipophilic Balance) convenablement sélectionnés, ainsi que des polymères associatifs non ioniques, anioniques, cationiques et amphotères. On peut citer par exemples les compositions pour la coloration des fibres kératiniques comprenant un colorant d'oxydation et un polymère épaississant comportant au moins une chaîne grasse et au moins un alcool gras mono ou polyglycérolé décrites dans le document US2001/023514.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il a déjà été proposé, notamment dans la demande de brevet allemand DE 30 16 008 des compositions pour la teinture d'oxydation des fibres kératiniques comprenant du 2-chloro 6-méthyl 3-amino phénol ou du 2-méthyl 6-chloro 3-amino phénol à titre de coupleur, en association avec des bases d'oxydation classiquement utilisées pour la teinture d'oxydation, telles que par exemple certaines para-phénylènediamines, du para-aminophénol ou des bases hétérocycliques.

La demande de brevet WO 96/15765 décrit des compositions pour la teinture d'oxydation des fibres kératiniques comprenant le 2-chlore 6-méthyl 3-amino phénol à titre de coupleur et le 2-(2,5-diaminophényl) éthanol à titre de base d'oxydation. La demande de brevet WO 96/15766 décrit des compositions pour la teinture d'oxydation des fibres kératiniques comprenant l'association spécifique du 2-chloro 6-méthyl 3-amino phénol à titre de coupleur et d'une base d'oxydation particulière choisie parmi les dérivés de para-aminophénol substitué en position 2 ou 3 comme par exemple le 3-méthyl 4-amino phénol, le 2-allyl 4-amino, phénol ou bien encore le 2-aminométhyl 4-amino phénol. Les documents DE 20118982 et WO 0141717 citent également à titre de coupleurs utilisables en coloration capillaire le 2-chloro 6-méthyl 3-amino phénol.

De telles compositions ne sont cependant pas entièrement satisfaisantes notamment du point de vue de la tenue des colorations obtenues vis-à-vis des diverses agressions que peuvent subir les cheveux, et en particulier vis-à-vis des shampooings, de la lumière, de la sueur et des déformations permanentes et du point de vue de la puissance et de la chromaticité des colorations obtenues.

Le but de la présente invention est de fournir de nouvelles compositions pour la teinture d'oxydation des fibres kératiniques ne présentant pas les inconvénients de celles de l'art antérieur. En particulier, le but de la présente invention est de fournir de nouvelles compositions conduisant à des colorations aux nuances variées, puissantes, chromatiques, esthétiques, peu sélectives et résistant bien aux diverses agressions que peuvent subir les cheveux tels que les shampooings, la lumière, la sueur et les déformations permanentes.

Ce but est atteint avec la présente invention qui a pour objet une composition pour la teinture d'oxydation des fibres kératiniques comprenant, dans un milieu de teinture approprié :
- au moins un coupleur choisi parmi le 2-chloro 6-méthyl 3-amino phénol et ses sels d'addition ;
- au moins une base d'oxydation ; et
- au moins un polymère associatif épaississant comportant au moins une chaîne grasse en C₈-C₃₀.

La composition de la présente invention permet en particulier d'obtenir une coloration des fibres kératiniques plus intense et plus chromatique, peu sélective et tenace.

L'invention a aussi pour objet un procédé de teinture d'oxydation des fibres kératiniques, et en particulier des fibres kératiniques humaines telles que les cheveux, mettant en oeuvre cette composition.

Un autre objet de l'invention est l'utilisation de la composition de la présente invention pour la teinture d'oxydation des fibres kératiniques, et en particulier des fibres kératiniques humaines telles que les cheveux.

Les bases d'oxydation pouvant être utilisées dans la composition tinctoriale conforme à l'invention sont de préférence choisies parmi les para-phénylènediamines, les bases doubles, les para-aminophénols, les ortho-aminophénols, les bases d'oxydation hétérocycliques et leurs sels d'addition.

Parmi les para-phénylènediamines utilisables à titre de base d'oxydation dans la composition tinctoriale conforme à l'invention, on peut notamment citer les composés de formule (I) suivante et leurs sels d'addition : 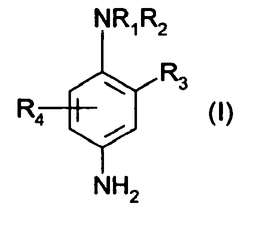 dans laquelle :
- R₁ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ; un radical alkyle en C₁-C₄ substitué par un groupement azoté ; un radical phényle ou un radical 4'-aminophényle ;
- R₂ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ou un radical alkyle en C₁-C₄ substitué par un groupement azoté ;
- R₁ et R₂ peuvent former ensemble, avec l'atome d'azote auquel ils sont rattachés, un hétérocycle comportant de 5 à 8 chaînons, éventuellement substitué par un ou plusieurs groupements choisis parmi les radicaux alkyle en C₁-C₄, amino, hydroxyalkyle en C₁-C₄ et trialkylammonium ;
- R₃ représente un atome d'hydrogène ; un atome d'halogène tel qu'un atome de chlore, de brome, d'iode ou de fluor ; un radical alkyle en C₁-C₄ ; un radical monohydroxyalkyle en C₁-C₄ ; un radical hydroxyalcoxy en C₁-C₄ ; un radical acétylaminoalcoxy en C₁-C₄ ; un radical mésylaminoalcoxy en C₁-C₄ ou un radical carbamoylaminoalcoxy en C₁-C₄ ;
- R₄ représente un atome d'hydrogène ; un atome d'halogène ou un radical alkyle en C₁-C₄.

Parmi les para-phénylènediamines de formule (I) ci-dessus, on peut plus particulièrement citer la para-phénylènediamine ; la para-toluylènediamine ; la 2-chloro para-phénylènediamine ; la 2,3-diméthyl para-phénylènediamine ; la 2,6-diméthyl para-phénylènediamine ; la 2,6-diéthyl para-phénylènediamine ; la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline ; la 4-N,N-bis-(β-hydroxyéthyl)amino 2-chloro aniline ; la 2-(β-hydroxyéthyl) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl) para-phénylènediamine ; le 2-(β-hydroxyéthyl)amino 5-amino toluène et leurs sels d'addition.

Parmi les para-phénylènediamines de formule (I) ci-dessus, on préfère tout particulièrement la para-phénylènediamine ; la para-toluylènediamine ; la 2-isopropyl para-phénylènediamine ; la 2-(β-hydroxyéthyl) para-phénylènediamine ; la 2-(β-hydroxyéthyloxy) para-phénylènediamine ; la 2,6-diméthyl para-phénylènediamine ; la 2,6-diéthyl para-phénylènediamine ; la 2,3-diméthyl para-phénylènediamine ; la N,N-bis-(β-hydroxyéthyl) para-phénylènediamine ; la 2-chloro para-phénylènediamine ; la 2-(β-acétylaminoéthyloxy) para-phénylènediamine et leurs sels d'addition.

Selon l'invention, on entend par bases doubles les composés comportant au moins deux noyaux aromatiques sur lesquels sont portés des groupements amino et / ou hydroxyle.

Parmi les bases doubles utilisables à titre de base d'oxydation dans la composition tinctoriale conforme à l'invention, on peut notamment citer les composés répondant à la formule (II) suivante, et leurs sels d'addition : 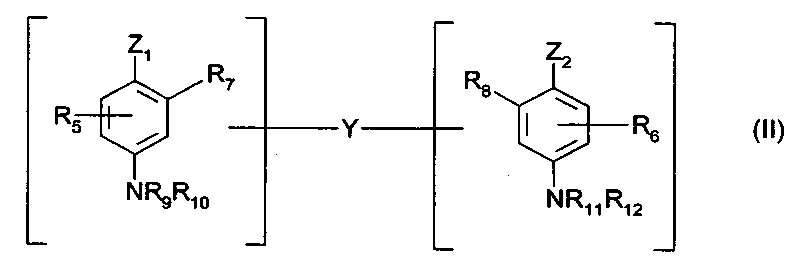 dans laquelle :
- Z₁ et Z₂, identiques ou différents, représentent un radical hydroxyle ou amino pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ; un atome d'halogène un radical alkyle en C₁-C₄ ; un radical monohydroxyalkyle en C₁-C₄ ; un radical polyhydroxyalkyle en C₂-C₄ ; un radical aminoalkyle en C₁-C₄ ou un bras de liaison Y ;
- R₇, R₈, R₉, R₁₀ R₁₁ et R₁₂, identiques ou différents, représentent un atome d'hydrogène ; un bras de liaison Y ; un radical alkyle en C₁-C₄ ou un radical monohydroxyalkyle en C₁-C₄ ;
étant entendu que les composés de formule (II) ne comportent qu'un seul bras de liaison Y par molécule.

Parmi les bases doubles de formule (II) ci-dessus dérivées de la para-phénylènediamine, on peut plus particulièrement citer le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4'-aminophényl) tétraméthylènediamine ; la N,N'-bis-(β-hydroxyéthyl) N,N'-bis-(4'-aminophényl) tétraméthylènediamine ; la N,N'-bis-(4'-méthyl-aminophényl) tétraméthylènediamine ; la N,N'-bis-(éthyl) N,N'-bis-(4'-amino, 3'-méthylphényl) éthylènediamine ; le 1,8-bis-(2',5'-diamino phénoxy)-3,6-dioxaoctane et leurs sels d'addition.

Parmi les bases doubles de formule (II) ci-dessus dérivées du para-aminophénol, on peut plus particulièrement citer le 4-amino 6-[(5'-amino 2'-hydroxy 3'-méthyl phényl)méthyl] 2-méthyl phénol ; le bis-(5'-amino 2'-hydroxy phényl) méthane et leurs sels d'addition.

Parmi ces bases doubles de formule (II), le N,N'-bis-(β-hydroxyéthyl) N,N'-bis-(4'-aminophényl) 1,3-diamino propanol ; le 1,8-bis-(2,5-diaminophénoxy)-3,5-dioxaoctane et leurs sels d'addition sont particulièrement préférés.

Parmi les para-aminophénols utilisables à titre de bases d'oxydation dans la composition tinctoriale conforme à l'invention, on peut notamment citer les composés répondant à la formule (III) suivante, et leurs sels d'addition : 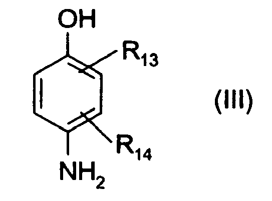 dans laquelle :
- R₁₃ représente un atome d'hydrogène ; un atome d'halogène ; un radical alkyle en C₁-C₄ ; un radical monohydroxyalkyle en C₁-C₄ ; un radical alcoxy(C₁-C₄)alkyle(C₁-C₄) ; un radical aminoalkyle en C₁-C₄ ou un radical hydroxyalkyl(C₁-C₄)aminoalkyle en C₁-C₄ ;
- R₁₄ représente un atome d'hydrogène ; un atome d'halogène ; un radical alkyle en C₁-C₄ ; un radical monohydroxyalkyle en C₁-C₄ ; un radical polyhydroxyalkyle en C₂-C₄ ; un radical aminoalkyle en C₁-C₄ ; un radical cyanoalkyle en C₁-C₄ ou un radical alcoxy(C₁-C₄)alkyle(C₁-C₄) ;
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol ; le 4-amino 3-méthyl phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthyl aminométhyl) phénol ; le 4-amino 2-fluoro phénol, le 4-amino 2-chloro phénol, le 4-amino 2,6-dichloro phénol et leurs sels d'addition.

Parmi les ortho-aminophénols utilisables à titre de base d'oxydation dans la composition tinctoriale conforme à l'invention, on peut plus particulièrement citer le 2-amino phénol ; le 2-amino 5-méthyl phénol ; le 2-amino 6-méthyl phénol ; le 5-acétamido 2-amino phénol et leurs sels d'addition.

Parmi les bases hétérocycliques utilisables à titre de base d'oxydation dans la composition tinctoriale conforme à l'invention, on peut plus particulièrement citer les dérivés pyridiniques, les dérivés pyrimidiniques, les dérivés pyrazoliques, les dérivés pyrazolo-pyrimidiniques et leurs sels d'addition.

Parmi les dérivés pyridiniques, on peut plus particulièrement citer les composés décrits par exemple dans les brevets GB 1 026 978 et GB 1 153 196, comme la 2,5-diamino pyridine ; la 2-(4-méthoxyphényl)amino 3-amino pyridine ; la 2,3-diamino 6-méthoxy pyridine ; la 2-(β-méthoxyéthyl)amino 3-amino 6-méthoxy pyridine ; la 3,4-diamino pyridine et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs sels d'addition.

Parmi les dérivés pyrimidiniques, on peut plus particulièrement citer les composés décrits par exemple dans les brevets DE 23 59 399, JP 88-169571, JP 05-63124, EP 0 770 375 ou la demande de brevet WO 96/15765 comme la 2,4,5,6-tétra-aminopyrimidine ; la 4-hydroxy 2,5,6-triaminopyrimidine ; la 2-hydroxy 4,5,6-triaminopyrimidine ; la 2,4-dihydroxy 5,6-diaminopyrimidine ; la 2,5,6-triaminopyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 38 43 892, DE 41 33 957 et les demandes de brevet WO 94/08969, WO 94/08970, FR-A-2 733 749 et DE 195 43 988 comme le 4,5-diamino 1-méthyl pyrazole ; le 4,5-diamino 1-(β-hydroxyéthyl) pyrazole ; le 3,4-diamino pyrazole ; le 4,5-diamino 1-(4'-chlorobenzyl) pyrazole ; le 4,5-diamino 1,3-diméthyl pyrazole ; le 4,5-diamino 3-méthyl 1-phényl pyrazole ; le 4,5-diamino 1-méthyl 3-phényl pyrazole ; le 4-amino 1,3-diméthyl 5-hydrazino pyrazole ; le 1-benzyl 4,5-diamino 3-méthyl pyrazole ; le 4,5-diamino 3-tert-butyl 1-méthyl pyrazole ; le 4,5-diamino 1-(β-hydroxyéthyl) 3-méthyl pyrazole ; le 4,5-diamino 1-tert-butyl 3-méthyl pyrazole ; le 4,5-diamino 1-éthyl 3-méthyl pyrazole ; le 4,5-diamino 1-éthyl 3-(4'-méthoxyphényl) pyrazole ; le 4,5-diamino 1-éthyl 3-hydroxyméthyl pyrazole ; le 4,5-diamino 3-hydroxyméthyl 1-méthyl pyrazole ; le 4,5-diamino 3-hydroxyméthyl 1-isopropyl pyrazole ; le 4,5-diamino 3-méthyl 1-isopropyl pyrazole ; le 4-amino 5-(2'-aminoéthyl)amino 1,3-diméthyl pyrazole ; le 3,4,5-triamino pyrazole ; le 1-méthyl 3,4,5-triamino pyrazole ; le 3,5-diamino 1-méthyl 4-méthylamino pyrazole ; le 3,5-diamino 4-(β-hydroxyéthyl)amino 1-méthyl pyrazole et leurs sels d'addition.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 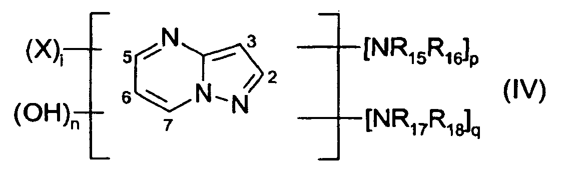 dans laquelle :
- R₁₅, R₁₆, R₁₇ et R₁₈, identiques ou différents, désignent un atome d'hydrogène ; un radical alkyle en C₁-C₄ ; un radical aryle ; un radical hydroxyalkyle en C₁-C₄ ; un radical polyhydroxyalkyle en C₂-C₄ ; un radical (C₁-C₄)alcoxy alkyle en C₁-C₄ ; un radical aminoalkyle en C₁-C₄, l'amine pouvant être protégée par un radical acétyle, uréido ou sulfonyle ; un radical (C₁-C₄)alkylamino alkyle en C₁-C₄ ; un radical di-[(C₁-C₄)alkyl] amino alkyle en C₁-C₄, les radicaux dialkyles pouvant former un cycle carboné ou un hétérocycle à 5 ou 6 chaînons ; un radical hydroxy(C₁-C₄)alkyl- ou di-[hydroxy(C₁-C₄) alkyl]-amino alkyle en C₁-C4 ;
- les radicaux X, identiques ou différents, désignent un atome d'hydrogène ; un radical alkyle en C₁-C₄ ; un radical aryle ; un radical hydroxyalkyle en C₁-C₄ ; un radical polyhydroxyalkyle en C₂-C₄ ; un radical amino alkyle en C₁-C₄ ; un radical (C₁-C₄)alkyl amino alkyle en C₁-C₄ ; un radical di-[(C₁-C₄)alkyl] amino alkyle en C₁-C₄, les dialkyles pouvant former un cycle carboné ou un hétérocycle à 5 ou 6 chaînons ; un radical hydroxy(C₁-C₄)alkyl ou di-[hydroxy(C₁-C₄ )alkyl]amino alkyle en C₁-C₄ ; un radical amino ; un radical (C₁-C₄)alkyl- ou di-[(C₁-C₄)alkyl]-amino ; un atome d'halogène ; un groupe acide carboxylique ; un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7).

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

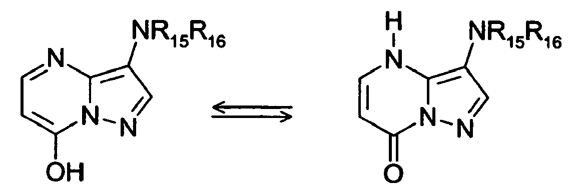

Parmi les pyrazolo-[1,5-a]-pyrimidines de formule (IV) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a]pyrimidin-7-yl)-(2-hydroxy-éthyl)-amino]-éthanol; le 2-[(7-Amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY ;
- R. Vishdu, H. Navedul, Indian J. Chem., 34b (6), 514, 1995 ;
- N.S. Ibrahim, K.U. Sadek, F.A. Abdel-Al, Arch. Pharm., 320, 240, 1987 ;
- R.H. Springer, M.B. Scholten, D.E. O'Brien, T. Novinson, J.P. Miller, R.K. ; Robins, J. Med. Chem., 25, 235, 1982 ;
- T. Novinson, R.K. Robins, T.R. Matthews, J. Med. Chem., 20, 296, 1977 ;
- US 3907799 ICN PHARMACEUTICALS.

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977 ;
- E. Alcade, J. De Mendoza, J.M. Marcia-Marquina, C. Almera, J. Elguero, J. Heterocyclic Chem., 11(3), 423, 1974 ;
- K. Saito, I. Hori, M. Higarashi, H. Midorikawa, Bull. Chem. Soc. Japan, 47(2), 476, 1974.

Les polymères associatifs épaississants utilisables selon l'invention sont des polymères hydrosolubles ou hydrodispersibles capables, dans un milieu aqueux, de s'associer réversiblement entre eux ou avec d'autres molécules.

Leur structure chimique comprend des zones hydrophiles et des zones hydrophobes caractérisées par au moins une chaîne grasse en C₈-C₃₀ et de préférence en C₁₀-C₃₀.

Ces polymères sont issus soit d'une polymérisation radicalaire ou d'une polycondensation à partir d'au moins un monomère dont l'un au moins possède une chaîne grasse en C₈-C₃₀, de préférence en C₁₀-C₃₀, soit du greffage sur un polymère, de préférence polyhydroxylé, d'un composé comportant au moins une chaîne grasse en C₈-C₃₀, de préférence en C₁₀-C₃₀.

On entend par "polymère épaississant" un polymère qui présente avantageusement en solution ou en dispersion, à 5 % de matière active dans l'eau, une viscosité mesurée au moyen du rhéomètre Rhéomat RM 180, à 25 °C, au moins égale à 500 cP, à un taux de cisaillement de 100 s⁻¹.

Les polymères associatifs épaississants selon l'invention peuvent être anioniques, cationiques, amphotères ou non ioniques. De préférence, les polymères associatifs sont anioniques, cationiques ou non ioniques.

Parmi les polymères associatifs de type anioniques, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci, et dont le motif éther d'allyle à chaîne grasse correspond au monomère de formule (V) suivante :

   CH₂ = C R' CH₂ O Bₙ R (V)

   dans laquelle:
   - R' désigne H ou CH₃ ;
   - B désigne le radical éthylèneoxy ;
   - n est nul ou désigne un entier allant de 1 à 100 ;
   - R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone.

   Des polymères associatifs anioniques de ce type sont décrits et préparés, selon un procédé de polymérisation en émulsion, dans le brevet EP-0 216479.
   Parmi ces polymères associatifs anioniques, on préfère particulièrement selon l'invention les polymères formés à partir de 20 à 60 % en poids d'acide acrylique et / ou d'acide méthacrylique, de 5 à 60 % en poids de (méth)acrylates d'alkyles inférieurs, de 2 à 50 % en poids d'éther d'allyle à chaîne grasse de formule (V) et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 ® et SALCARE SC90 ® qui sont des émulsions aqueuses à 30 % d'un terpolymère réticulé d'acide méthacrylique, d'acrylate d'éthyle et de steareth-10-allyl éther (40 / 50 / 10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (VI) suivante : 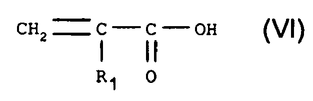 dans laquelle R₁ désigne H ou CH₃ ou C₂H₅, c'est-à-dire des motifs acide acrylique, acide méthacrylique ou acide éthacrylique, et dont le motif hydrophobe de type ester d'alkyle (C₁₀-C₃₀) d'acide carboxylique insaturé correspond au monomère de formule (VII) suivante 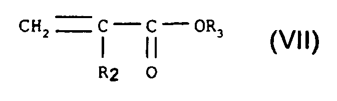 dans laquelle :
   - R₂ désigne H ou CH₃ ou C₂H₅, c'est-à-dire des motifs acrylates, méthacrylates ou éthacrylates, et de préférence H (motifs acrylates) ou CH₃ (motifs méthacrylates) ;
   - R₃ désignant un radical alkyle en C₁₀-C₃₀, et de préférence en C₁₂-C₂₂.
      Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
      Des polymères anioniques de ce type sont par exemple décrits et préparés, selon les brevets US-3 915 921 et 4 509 949.
      Parmi ce type de polymères associatifs anioniques, on utilisera plus particulièrement des polymères formés à partir d'un mélange de monomères comprenant :
      (i) essentiellement de l'acide acrylique ;
      (ii) un ester de formule (VII) décrite ci-dessus et dans laquelle R₂ désigne H ou CH₃, R₃ désignant un radical alkyle ayant de 12 à 22 atomes de carbone ;
      (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
      Parmi ce type de polymères associatifs anioniques, on utilisera plus particulièrement ceux constitués de 95 à 60 % en poids d'acide acrylique (motif hydrophile), de 4 à 40 % en poids d'acrylate d'alkyle en C₁₀-C₃₀ (motif hydrophobe), et de 0 à 6 % en poids de monomère polymérisable réticulant, ou bien ceux constitués de 98 à 96 % en poids d'acide acrylique (motif hydrophile), de 1 à 4 % en poids d'acrylate d'alkyle en C₁₀-C₃₀ (motif hydrophobe), et de 0,1 à 0,6 % en poids de monomère polymérisable réticulant tel que ceux décrits précédemment.
      Parmi lesdits polymères ci-dessus, on préfère tout particulièrement selon la présente invention, les produits vendus par la société GOODRICH sous les dénominations commerciales PEMULEN TR1 ®, PEMULEN TR2 ®, CARBOPOL 1382 ®, et encore plus préférentiellement le PEMULEN TR1 ®, et le produit vendu par la société S.E.P.P.I.C. sous la dénomination COATEX SX ®.
- (III) les terpolymères d'anhydride maléique / α-oléfine en C₃₀-C₃₈ / maléate d'alkyle tel que le produit (copolymère anhydride maléique / α-oléfine en C₃₀-C₃₈ / maléate d'isopropyle) vendu sous le nom PERFORMA V 1608 ® par la société NEWPHASE TECHNOLOGIES.
- (IV) les terpolymères acryliques comprenant :
   (a) environ 20 % à 70 % en poids d'un acide carboxylique à insaturation α,β-monoéthylénique ;
   (b) environ 20 à 80 % en poids d'un monomère à insaturation α,β-monoéthylénique non-tensio-actif différent de (a) ;
   (c) environ 0,5 à 60 % en poids d'un mono-uréthane non-ionique qui est le produit de réaction d'un tensio-actif monohydrique avec un monoisocyanate à insaturation monoéthylénique ;
   tels que ceux décrits dans la demande de brevet EP-A-0 173 109 et plus particulièrement celui décrit dans l'exemple 3, à savoir, un terpolymère acide méthacrylique / acrylate de méthyle / diméthyl métaisopropényl benzyl isocyanate d'alcool béhényle éthoxylé (40 OE) en dispersion aqueuse à 25 %.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.
   Préférentiellement, ces composés comprennent également comme monomère un ester d'acide carboxylique à insaturation α,β-monoéthylénique et d'alcool en C₁-C₄.
   A titre d'exemple de ce type de composé, on peut citer l'ACULYN 22 ® vendu par la société ROHM et HAAS, qui est un terpolymère acide méthacrylique / acrylate d'éthyle / méthacrylate de stéaryle oxyalkyléné.
- (VI) les polyuréthanes associatifs à caractère anionique tels que le VISCOPHOBE DB 1000 de la société UNION CARBIDE.

Parmi les polymères associatifs de type cationique, on peut citer parmi eux :
- (I) les polyuréthanes associatifs cationiques dont la famille a été décrite par la demanderesse dans la demande de brevet français FR 0 009 609. Elle peut être représentée par la formule générale (VIII) suivante :

   R-X-(P)ₙ-[L-(Y)ₘ]ᵣ-L'-(P')ₚ-X'-R' (VIII)
dans laquelle :
- R et R', identiques ou différents, représentent un groupement hydrophobe ou un atome d'hydrogène ;
- X et X', identiques ou différents, représentent un groupement comportant une fonction amine portant ou non un groupement hydrophobe, ou encore le groupement L" ;
- L, L' et L", identiques ou différents, représentent un groupement dérivé d'un diisocyanate ;
- P et P', identiques ou différents, représentent un groupement comportant une fonction amine portant ou non un groupement hydrophobe ;
- Y représente un groupement hydrophile ;
- r est un nombre entier compris entre 1 et 100, de préférence entre 1 et 50 et en particulier entre 1 et 25 ;
- n, m, et p valent chacun indépendamment des autres entre 0 et 1000 ; la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (VIII) décrite ci-dessus et dans laquelle :
- R et R' représentent tous les deux indépendamment un groupement hydrophobe ;
- X et X' représentent chacun un groupe L" ;
- n et p valent entre 1 et 1000 ; et
- L, L', L", P, P', Y et m ont la signification indiquée ci-dessus.

Une autre famille préférée de polyuréthanes associatifs cationiques est celle correspondant à la formule (VIII) ci-dessus dans laquelle :
- R et R' représentent tous les deux indépendamment un groupement hydrophobe ;
- X et X' représentent chacun un groupe L" ;
- n et p valent 0 ; et
- L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (VIII) ci-dessus dans laquelle :
- R et R' représentent tous les deux indépendamment un groupement hydrophobe ;
- X et X' représentent tous les deux indépendamment un groupement comportant une amine quaternaire ;
- n et p valent zéro ; et
- L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et / ou X' désignent un groupement comportant une amine tertiaire ou quaternaire, X et / ou X' peuvent représenter l'une des formules suivantes : 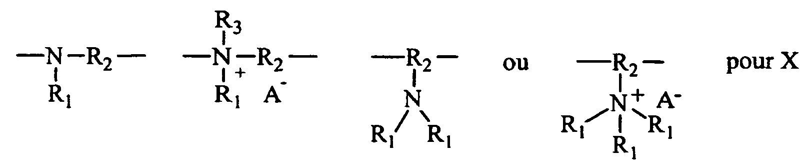 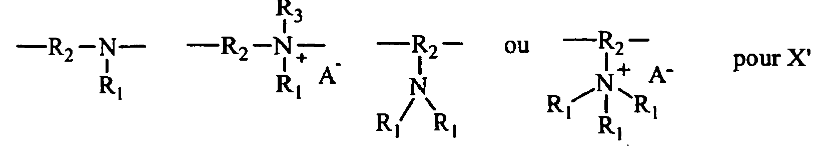 dans lesquelles :
- R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
- R₁ et R₃, identiques ou différents, désignent un radical alkyle ou alcényle en C₁-C₃₀. linéaire ou ramifié, un radical aryle, l'un au moins des atomes de carbone pouvant être remplacé par un hétéroatome choisi parmi N, S, O, P ;
- A⁻ est un contre-ion physiologiquement acceptable.

Les groupements L, L' et L" représentent un groupe de formule : 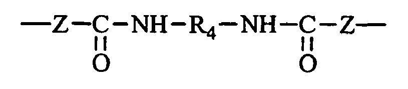 dans laquelle :
- Z représente -O-, -S- ou -NH- ; et
- R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 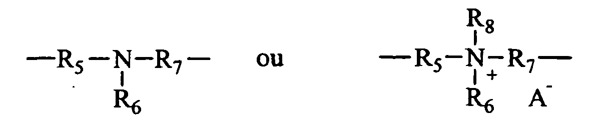 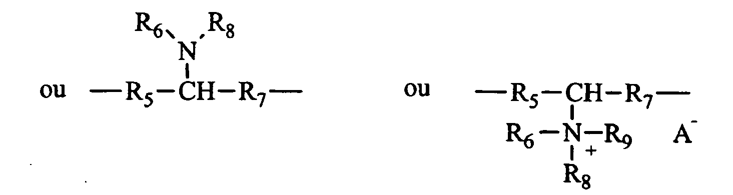 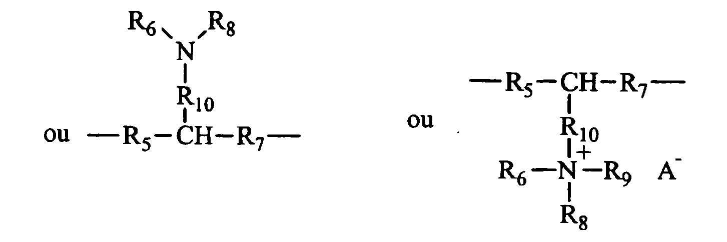 dans lesquelles :
- R₅ et R₇ ont les mêmes significations que R₂ défini précédemment ;
- R₆, R₈ et R₉ ont les mêmes significations que R₁ et R₃ définis précédemment ;
- R₁₀ représente un groupe alkylène, linéaire ou ramifié, éventuellement insaturé et pouvant contenir un ou plusieurs hétéroatomes choisis parmi N, O, S et P ; et
- A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (VIII) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (VIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH,

ou

HZ-(P')p-ZH

dans lesquelles Z, P, P', n et p sont tels que définis plus haut.

A titre d'exemple de composé à fonction amine, on peut citer la N-méthyldiéthanolamine ; la N-tert-butyl-diéthanolamine ; la N-sulfoéthyldiéthanolamine.

Le deuxième composé entrant dans la préparation du polyuréthane de formule (VIII) est un diisocyanate correspondant à la formule:

O=C=N-R₄-N=C=O

dans laquelle R₄ est défini plus haut.

A titre d'exemple, on peut citer le méthylènediphényl-diisocyanate ; le méthylènecyclohexanediisocyanate ; l'isophorone-diisocyanate ; le toluènediisocyanate ; le naphtalènediisocyanate ; le butanediisocyanate ; l'hexanediisocyanate.

Un troisième composé entrant dans la préparation du polyuréthane de formule (VIII) est un composé hydrophobe destiné à former les groupes hydrophobes terminaux du polymère de formule (VIII).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné -hydroxyle.

Le groupe hydrophobe du polyuréthane de formule (VIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate, etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (VIII) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.
   Les dérivés de cellulose quaternisée sont en particulier,
   - les celluloses quaternisées modifiées par des groupements comportant au moins une chaîne grasse, tels que les groupes alkyle, arylalkyle, alkylaryle comportant au moins 8 atomes de carbone ou des mélanges de ceux-ci,
   - les hydroxyéthylcelluloses quaternisées modifiées par des groupements comportant au moins une chaîne grasse, tels que les groupes alkyle, arylalkyle, alkylaryle comportant au moins 8 atomes de carbone ou des mélanges de ceux-ci.
      Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
      On peut indiquer comme exemples d'alkylhydroxyéthylcelluloses quatemisées à chaînes grasses en C₈-C₃₀, les produits QUATRISOFT LM 200 ®, QUATRISOFT LM-X 529-18-A ®, QUATRISOFT LM-X 529-18B ® (alkyle en C₁₂) et QUATRISOFT LM-X 529-8 ® (alkyle en C₁₈) commercialisés par la société AMERCHOL et les produits CRODACEL QM ®, CRODACEL QL ® (alkyle en C₁₂) et CRODACEL QS ® (alkyle en C₁₈) commercialisés par la société CRODA.
- (III) Les polyvinyllactames cationiques dont la famille a été décrite par la demanderesse dans la demande de brevet français FR 0 101 106.
Lesdits polymères comprennent :
(a) au moins un monomère de type vinyl lactame ou alkylvinyllactame;
(b) au moins un monomère de structures (IX) ou (X) suivantes : 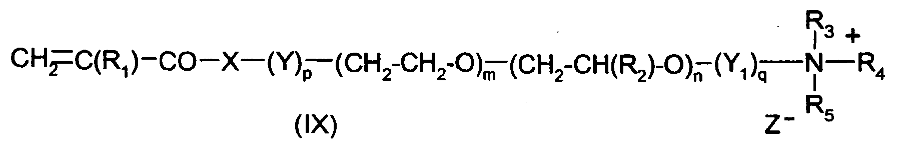 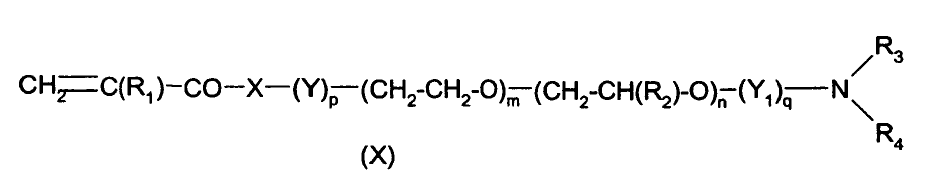
dans lesquelles :
- X désigne un atome d'oxygène ou un radical NR₆ ;
- R₁ et R₆ désignent, indépendamment l'un de l'autre, un atome d'hydrogène ou un radical alkyl linéaire ou ramifié en C₁-C₅ ;
- R₂ désigne un radical alkyle linéaire ou ramifié en C₁-C₄ ;
- R₃, R₄ et R₅ désignent, indépendamment l'un de l'autre, un atome d'hydrogène, un radical alkyle linéaire ou ramifié en C₁-C₃₀ ou un radical de formule (XI) : 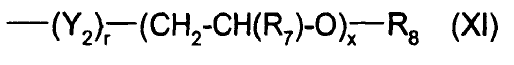
dans laquelle :
- Y , Y₁ et Y₂ désignent, indépendamment l'un de l'autre, un radical alkylène linéaire ou ramifié en C₂-C₁₆ ;
- R₇ désigne un atome d'hydrogène, ou un radical alkyle linéaire ou ramifié en C₁-C₄ ou un radical hydroxyalkyle linéaire ou ramifié en C₁-C₄;
- R₈ désigne un atome d'hydrogène ou un radical alkyle linéaire ou ramifié en C₁-C₃₀ ;
- p, q et r désignent, indépendamment l'un de l'autre, soit la valeur zéro, soit la valeur 1 ;
- m et n désignent, indépendamment l'un de l'autre, un nombre entier allant de 0 à 100 ;
- x désigne un nombre entier allant de 1 à 100 ;
- Z désigne un anion d'acide organique ou minéral ;
sous réserve que :
- l'un au moins des substituants R₃, R₄, R₅ ou R₈ désigne un radical alkyle linéaire ou ramifié en C₉-C₃₀ ;
- si m ou n est différent de zéro, alors q est égal à 1 ;
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence, le contre ion Z⁻ des monomères de formule (I) est choisi parmi les ions halogénures, les ions phosphates, l'ion méthosulfate, l'ion tosylate.

De préférence, R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (IX) pour laquelle, encore plus préférentiellement, m et n sont égaux à zéro.

Le monomère vinyl lactame ou alkylvinyllactame est de préférence un composé de structure (XII) : 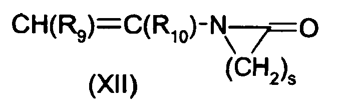 dans laquelle :
- s désigne un nombre entier allant de 3 à 6 ;
- R₉ désigne un atome d'hydrogène ou un radical alkyle en C₁-C₅ ;
- R₁₀ désigne un atome d'hydrogène ou un radical alkyle en C₁-C₅ ; sous réserve que l'un au moins des radicaux R₉ et R₁₀ désigne un atome d'hydrogène.

Encore plus préférentiellement, le monomère (XII) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
(a) un monomère de formule (XII) ;
(b) un monomère de formule (IX) dans laquelle p = 1, q = 0, R₃ et R₄ désignent, indépendamment l'un de l'autre, un atome d'hydrogène ou un radical alkyle en C₁-C₅ et R₅ désigne un radical alkyle en C₉-C₂₄ ; et
(c) un monomère de formule (X) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95 % de monomère (a), 0,1 à 55 % de monomère (c) et 0,25 à 50 % de monomère (b).

De tels polymères sont décrits dans la demande de brevet WO 00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium ; les terpolymères vinylpyrrolidone / diméthylaminopropylméthacrylamide / tosylate de cocoyldiméthylméthacrylamido propylammonium ; les terpolymères vinylpyrrolidone / diméthylaminopropyl méthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les polymères associatifs amphotères sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles % et plus particulièrement encore 1,5 à 6 moles %, par rapport au nombre total de moles de monomères.

Les polymères associatifs amphotères préférés selon l'invention comprennent, ou sont préparés en copolymérisant :
1) au moins un monomère de formule (XIII) ou (XIV): 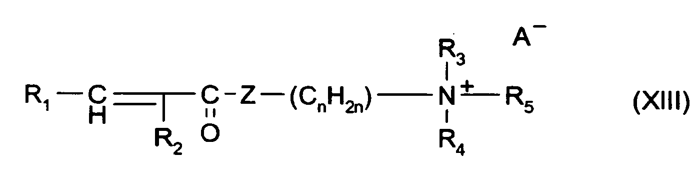 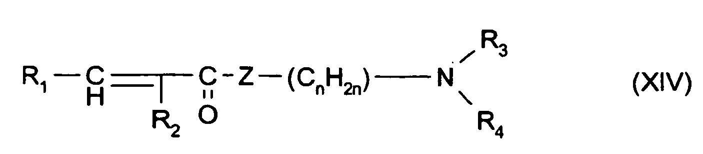 dans lesquelles :
   - R₁ et R₂, identiques ou différents, représentent un atome d'hydrogène ou un radical méthyle ;
   - R₃, R₄ et R₅, identiques ou différents, représente un radical alkyle linéaire ou ramifié ayant de 1 à 30 atomes de carbone ;
   - Z représente un groupe NH ou un atome d'oxygène ;
   - n est un nombre entier de 2 à 5 ;
      - A- est un anion issu d'un acide organique ou minéral, tel qu'un anion méthosulfate ou un halogénure tel que chlorure ou bromure ;
2) au moins un monomère de formule (XV)

   R₆―CH = CR₇―COOH (XV)

   dans laquelle :
   - R₆ et R₇, identiques ou différents, représentent un atome d'hydrogène ou un radical méthyle ; et
3) au moins un monomère de formule (XVI) :

   R₆―CH = CR₇―COXR₈ (XVI)
dans laquelle :
- R₆ et R₇, identiques ou différents, représentent un atome d'hydrogène ou un radical méthyle ;
- X désigne un atome d'oxygène ou d'azote ; et
- R₈ désigne un radical alkyle linéaire ou ramifié ayant de 1 à 30 atomes de carbone ;
l'un au moins des monomères de formule (XIII), (XIV) ou (XV) comportant au moins une chaîne grasse.

Les monomères de formule (XIII) et (XIV) de la présente invention sont choisis, de préférence, dans le groupe constitué par :
- le diméthylaminoéthylméthacrylate, le diméthylaminoéthylacrylate ;
- le diéthylaminoéthylméthacrylate, le diéthylaminoéthylacrylate ;
- le diméthylaminopropylméthacrylate, le diméthylaminopropylacrylate ;
- le diméthylaminopropylméthacrylamide, le diméthylaminopropylacrylamide ; ces monomères étant éventuellement quaternisés, par exemple par un halogénure d'alkyle en C₁-C₄ ou un sulfate de dialkyle en C₁-C₄.

Plus particulièrement, le monomère de formule (XIII) est choisi parmi le chlorure d'acrylamidopropyl triméthyl ammonium et le chlorure de méthacrylamidopropyl triméthyl ammonium.

Les monomères de formule (XIV) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XIV) est l'acide acrylique.

Les monomères de formule (XIV) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et / ou quaternisés.

Le rapport du nombre de charges cationiques / charges anioniques est de préférence égal à environ 1.

Les polymères associatifs amphotères selon l'invention comprennent de préférence de 1 à 10 moles % du monomère comportant une chaîne grasse (monomère de formule (XIII), (XIV) ou (XV)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO 98/44012.

Parmi les polymères associatifs amphotères selon l'invention, on préfère les terpolymères acide acrylique / chlorure de (méth)acrylamidopropyl triméthyl ammonium / méthacrylate de stéaryle.

Selon l'invention, les polymères associatifs non ioniques sont choisis de préférence parmi :
- (1 ) les celluloses modifiées par des groupements comportant au moins une
   chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS ® (alkyles en C₁₆) vendu par la société AQUALON, ou le produit BERMOCOLL EHM 100 ® vendu par la société BEROL NOBEL ;
   - celles modifiées par des groupes polyalkylène glycol éther d'alkyl phénol, tel que le produit AMERCELL POLYMER HM-1500 ® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22 ® (chaîne alkyle en C₂₂) vendu par la société LAMBERTI, les produits RE210-18 ® (chaîne alkyle en C₁₄) et RE205-1 ® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216 ® ou GANEX V216 ® (copolymère vinylpyrrolidone / hexadécène) vendu par la société I.S.P. ;
   - les produits ANTARON V220 ® ou GANEX V220 ® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle / acrylate de stéaryle oxyéthyléné vendu par la société GOLDSCHMIDT sous la dénomination ANTIL 208 ®.
- (5) les copolymères de méthacrylates ou d'acrylates hydrophiles et de monomères hydrophobes comportant au moins une chaîne grasse tels que par exemple le copolymère méthacrylate de polyéthylèneglycol / méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et / ou des enchaînements cycloaliphatiques et / ou aromatiques.
- (7) les polymères à squelette aminoplaste éther possédant au moins une chaîne grasse, tels que les composés PURE THIX ® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205 ® à fonction urée vendu par la société RHEOX ou encore les Rhéolates ® 208 , 204 ou 212, ainsi que l'Acrysol RM 184 ®.

On peut également citer le produit ELFACOS T210 ® à chaîne alkyle en C₁₂₋₁₄ et le produit ELFACOS T212 ® à chaîne alkyle en C₁₈ de chez AKZO.

Le produit DW 1206B ® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate ® 255, le Rhéolate ® 278 et le Rhéolate ® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46 ® et Aculyn 44 ® [l'ACULYN 46 ® est un polycondensat de polyéthylèneglycol à 150 ou 180 moles d'oxyde d'éthylène, d'alcool stéarylique et de méthylène bis(4-cyclohexyl-isocyanate) (SMDI), à 15 % en poids dans une matrice de maltodextrine (4 %) et d'eau (81 %) ; l'ACULYN 44 ® est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

Dans une composition préférée selon la présente invention, les polymères associatifs épaississants sont choisis parmi les polymères associatifs non ioniques ou cationiques et plus particulièrement encore parmi les polyuréthanes polyéthers à séquences hydrophiles et hydrophobes, les polymères à squelette aminoplaste éther comportant au moins une chaîne grasse, les polyuréthanes associatifs cationiques, les dérivés de cellulose quaternisée comportant au moins une chaîne grasse, et les polyvinyllactames cationiques.

Dans une composition encore plus particulièrement préférée selon l'invention, le polymère associatif épaississant est choisi parmi les alkyl(C₈-C₃₀)hydroxyéthylcelluloses quaternisées et notamment la laurylhydroxyéthylcellulose quaternisée.

La composition selon l'invention peut contenir en plus du 2-chlore 6-méthyl 3-amino phénol un ou plusieurs coupleurs additionnels choisis parmi les coupleurs conventionnellement utilisés pour la teinture d'oxydation des fibres kératiniques. Parmi ces coupleurs, on peut notamment citer les méta-phénylènediamines, les méta-aminophénols différents du 2-chloro 6-méthyl 3-amino phénol, les méta-diphénols, les coupleurs naphtaléniques, les coupleurs hétérocycliques ainsi que leur sels d'addition.

A titre d'exemple, on peut citer le 2-méthyl 5-amino phénol, le 5-N-(ß-hydroxyéthyl)amino 2-méthyl phénol ; le 3-amino phénol ; le 1,3-dihydroxy benzène ; le 1,3-dihydroxy 2-méthyl benzène ; le 4-chloro 1,3-dihydroxy benzène ; le 2,4-diamino 1-(ß-hydroxyéthyloxy) benzène ; le 2-amino 4-(ß-hydroxyéthylamino) 1-méthoxy benzène ; le 1,3-diamino benzène ; le 1,3-bis-(2,4-diaminophénoxy) propane ; la 3-uréido aniline ; le 3-uréido 1-benzène ; le sésamol ; le 1-ß-hydroxyéthylamino-3,4-méthylènedioxy benzène ; l'α-naphtol ; le 2 méthyl-1-naphtol ; le 6-hydroxy indole ; le 4-hydroxy indole ; le 4-hydroxy N-méthyl indole ; la 2-amino-3-hydroxy pyridine ; la 6- hydroxy benzomorpholine ; la 3,5-diamino-2,6-diméthoxy pyridine ; le 1-N-(ß-hydroxyéthyl)amino-3,4-méthylènedioxy benzène ; le 2,6-bis-(ß-hydroxyéthylamino) toluène et leurs sels d'addition.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

Les polymères associatifs non ioniques, anioniques, cationiques ou amphotères sont utilisés de préférence en une quantité pouvant varier d'environ 0,01 à 10 % en poids environ du poids total de la composition tinctoriale. Plus préférentiellement, cette quantité varie d'environ 0,1 à 5 % en poids, et encore plus particulièrement d'environ 0,5 à 3 % en poids.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre comprendre un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids environ par rapport au poids total de la composition tinctoriale, et de préférence entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques autres que les polymères associatifs épaississants anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus peuvent être présents en quantité comprise pour chacun d'eux entre 0,001 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XVI) suivante : 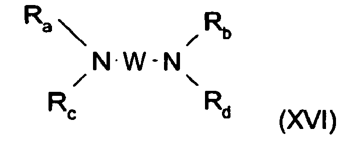 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel la composition conforme à l'invention telle que définie précédemment est appliquée sur les fibres kératiniques, et la couleur est révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le comprenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale conforme à l'invention et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

Les compositions tinctoriales ci-dessous sont préparées :

| **Exemple** | **1** | **2** | **3** | **4** |
|---|---|---|---|---|
| 2-chloro 6-méthyl 3-amino phénol | 1,26 g | 1,26 g | 1,26 g | 1,26 g |
| Para-aminophénol | 0,872 g | | | |
| 3-méthyl 4-amino phénol | | | | 0,984 g |
| Para-phénylènediamine | | | 0,864 g | |
| Para-toluènediamine | | 0,976 g | | |
| Aculyn 44 ® ou Aculyn 46 ® commercialisé par Rohm & Haas | 4 g M.A. | | | |
| Pemulen TR1 ® commercialisé par Goodrich | | 3,8 g M.A. | | |
| Quadrisoft LM 200 ® commercialisé par Amerchol | | | 3,8 g M.A. | 3,8 g M.A. |
| Support de teinture | (*) | (*) | (*) | (*) |
| Eau déminéralisée q.s.p. | 100 g | 100 g | 100 g | 100 g |

| | | | | |
|---|---|---|---|---|
| (*) : support de teinture commun | | | | |

| | |
|---|---|
| Mélange d'alcools linéaires en C₁₈ à C₂₄ (C₁₈ / C₂₀ / C₂₂ / C₂₄ : 7 / 58 / 30 / 6 - teneur en alcool > 95 %) | 3,0 g |
| Mélange d'alcools linéaires en C₁₈ à C₂₄ (C₁₈ / C₂₀ / C₂₂ / C₂₄ : 7 / 58 / 30 / 6 - teneur en alcool > 95 %) oxyéthylénés (30 OE) | 1,0 g |
| Alcool stéarylique oxyéthyléné (2 OE) | 4,5 g |
| Alcool stéarylique oxyéthyléné (21 OE) | 1,75 g |
| Polymère cationique W | 4,0 g M.A. |
| Acide oléique | 2,6 g |
| Acide polyacrylique réticulé (Carbopol 980 de Goodrich) | 0,6 g |
| Monoisopropanolamide d'acides de coprah | 3,0 g |
| Hexylène glycol | 6,0 g |
| Métabisulfite de sodium | 0,71 g |
| EDTA | 0,2 g |
| Terbutyl hydroquinone | 0,3 g |
| Monoéthanolamine | 1,0 g |
| Ammoniaque à 20% de NH₃ | 11,0 g |
| Parfum | q.s. |

La composition oxydante ci-dessous est préparée :

| | |
|---|---|
| Alcool gras | 2,3 g |
| Alcool gras oxyéthyléné | 0,6 g |
| Amide grasse | 0,9 g |
| Glycérine | 0,5 g |
| Peroxyde d'hydrogène | 7,5 g |
| Parfum | q.s. |
| Agent de pH, pH = 3 | q.s. |
| Eau déminéralisée q.s.p. | 100 g |

La composition tinctoriale est mélangée extemporanément avec une fois et demi son poids de composition oxydante.
Le mélange ainsi réalisé est appliqué sur des mèches de cheveux gris à 90% de blancs naturels, à raison de 30 g pour 3 g de cheveux, pendant 30 minutes. Les cheveux sont ensuite rincés, lavés avec un shampooing standard et séchés.
La coloration capillaire est évaluée de manière visuelle. Les nuances obtenues figurent dans le tableau ci-dessous.

| **Exemple** | **1** | **2** | **3** | **4** |
|---|---|---|---|---|
| Hauteur de ton | Blond | Blond foncé | Blond foncé | Blond |
| Reflet | Rouge cuivré | Violacé | Violine | Rouge |

## Revendications

1. Composition pour la teinture d'oxydation des fibres kératiniques comprenant, dans un milieu de teinture approprié :
- au moins un coupleur choisi parmi le 2-chloro 6-méthyl 3-amino phénol et ses sels d'addition ;
- au moins une base d'oxydation ; et
- au moins un polymère associatif épaississant comportant au moins une chaîne grasse en C₈-C₃₀.

2. Composition selon la revendication 1, dans laquelle la ou les bases d'oxydation sont choisies parmi les para-phénylènediamines, les bases doubles, les para-aminophénols, les ortho-aminophénols, les bases d'oxydation hétérocycliques ainsi que leurs sels d'addition.

3. Composition selon la revendication 2, dans laquelle les para-phénylènediamines sont choisies parmi les composés de formule (I) suivante et leurs sels d'addition : 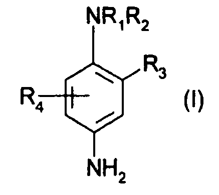 dans laquelle :
- R₁ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ; un radical alkyle en C₁-C₄ substitué par un groupement azoté ; un radical phényle ou un radical 4'-aminophényle ;
- R₂ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ou un radical alkyle en C₁-C₄ substitué par un groupement azoté ;
- R₁ et R₂ peuvent former ensemble, avec l'atome d'azote auquel ils sont rattachés, un hétérocycle comportant de 5 à 8 chaînons, éventuellement substitué par un ou plusieurs groupements choisis parmi les radicaux alkyle en C₁-C₄, amino, hydroxyalkyle en C₁-C₄ et trialkylammonium ;
- R₃ représente un atome d'hydrogène ; un atome d'halogène tel qu'un atome de chlore, de brome, d'iode ou de fluor ; un radical alkyle en C₁-C₄; un radical monohydroxyalkyle en C₁-C₄ un radical hydroxyalcoxy en C₁-C₄ ; un radical acétylaminoalcoxy en C₁-C₄ ; un radical mésylaminoalcoxy en C₁-C₄ ou un radical carbamoylaminoalcoxy en C₁-C₄ ;
- R₄ représente un atome d'hydrogène ; un atome d'halogène ou un radical alkyle en C₁-C₄.

4. Composition selon la revendication 3, dans laquelle les para-phénylènediamines de formule (I) sont choisies parmi la para-phénylènediamine ; la para-toluylènediamine ; la 2-chloro para-phénylènediamine ; la 2,3-diméthyl para-phénylènediamine : la 2,6-diméthyl para-phénylènediamine ; la 2,6-diéthyl para-phénylènediamine : la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline ; la 4-N,N-bi-(β-hydroxyéthyl)amino 2-chloro aniline ; la 2-(β-hydroxyéthyl) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl)amino para-phénylènediamine et leurs sels d'addition.

5. Composition selon l'une quelconque des revendications 2 à 4, dans laquelle les bases doubles sont choisies parmi les composés répondant à la formule (II) suivante, et leurs sels d'addition : 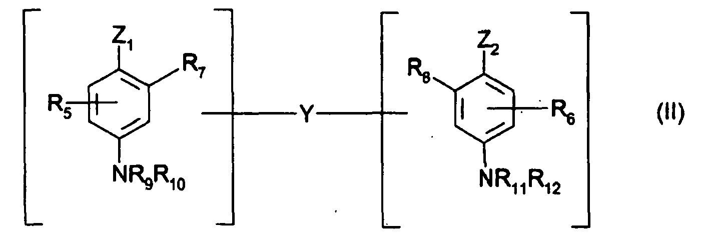 dans laquelle :
- Z₁ et Z₂, identiques ou différents, représentent un radical hydroxyle ou amino pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ; un atome d'halogène ; un radical alkyle en C₁-C₄ un radical monohydroxyalkyle en C₁-C₄ ; un radical polyhydroxyalkyle en C₂-C₄ ; un radical aminoalkyle en C₁-C₄ ou un bras de liaison Y ;
- R₇, R₈, R₉, R₁₀ R₁₁ et R₁₂, identiques ou différents, représentent un atome d'hydrogène ; un bras de liaison Y ; un radical alkyle en C₁-C₄ ou un radical monohydroxyalkyle en C₁-C₄ ;
étant entendu que les composés de formule (II) ne comportent qu'un seul bras de liaison Y par molécule.

6. Composition selon la revendication 5, dans laquelle les bases doubles de formule (II) sont choisies parmi le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4-aminophényl) tétraméthylènediamine ; la N,N'-bis-(β-hydroxyéthyl) N,N'-bis-(4-aminophényl) tétraméthylènediamine ; la N,N'-bis-(4-méthyl-aminophényl) tétraméthylènediamine ; la N,N'-bis-(éthyl) N,N'-bis-(4'-amino, 3'-méthylphényl) éthylènediamine ; le 1,8-bis-(2,5-diamino phénoxy)-3,5-dioxaoctane ; le 4-amino 6-[(5'-amino 2'-hydroxy 3'-méthyl phényl)méthyl] 2-méthyl phénol ; le bis-(5-amino 2-hydroxy phényl) méthane et leurs sels d'addition.

7. Compositions selon l'une quelconque des revendications 2 à 6, dans laquelle les para-aminophénols sont choisis parmi les composés répondant à la formule (III) suivante et leurs sels d'addition : 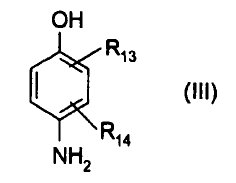 dans laquelle :
- R₁₃ représente un atome d'hydrogène ; un atome d'halogène ; un radical alkyle en C₁-C₄ ; un radical monohydroxyalkyle en C₁-C₄ un radical alcoxy(C₁-C₄)alkyle(C₁-C₄) ; un radical aminoalkyle en C₁-C₄ ou un radical hydroxyalkyl(C₁-C₄)aminoalkyle en C₁-C₄ ;
- R₁₄ représente un atome d'hydrogène ; un atome d'halogène ; un radical alkyle en C₁-C₄ : un radical monohydroxyalkyle en C₁-C₄ ; un radical polyhydroxyalkyle en C₂-C₄ ; un radical aminoalkyle en C₁-C₄ : un radical cyanoalkyle en C₁-C₄ ou un radical alcoxy(C₁-C₄)alkyle(C₁-C₄) ;
étant entendu qu'au moins un des radicaux R₁₃ ou R₁₄ représente un atome d'hydrogène.

8. Compositions selon la revendication 7, dans laquelle les para-aminophénols sont choisis parmi le para-aminophénol ; le 4-amino 3-méthyl phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthyl aminométhyl) phénol ; le 4-amino 2-fluoro phénol ; le 4-amino 2-chloro phénol ; le 4-amino 2,6-dichloro phénol et leurs sels d'addition.

9. Composition selon l'une quelconque des revendications 2 à 8, dans laquelle les ortho-aminophénols sont choisis parmi le 2-amino phénol ; le 2-amino 5-méthyl phénol ; le 2-amino 6-méthyl phénol ; le 5-acétamido 2-amino phénol et leurs sels d'addition.

10. Composition selon l'une quelconque des revendications 2 à 9, dans laquelle les bases hétérocycliques sont choisies parmi les dérivés pyridiniques, les dérivés pyrimidiniques, les dérivés pyrazoliques, les dérivés pyrazolo-pyrimidiniques et leurs sels d'addition.

11. Composition selon l'une quelconque des revendications précédentes, dans laquelle les polymères associatifs comprennent des zones hydrophiles et des zones hydrophobes **caractérisées par** au moins une chaîne grasse en C₈-C₃₀.

12. Composition selon l'une quelconque des revendications précédentes, dans laquelle les polymères associatifs sont issus soit d'une polymérisation radicalaire ou d'une polycondensation à partir d'au moins un monomère dont l'un au moins possède une chaîne grasse en C₈-C₃₀, soit du greffage sur un polymère d'un composé comportant au moins une chaîne grasse en C₈-C₃₀.

13. Composition selon l'une quelconque des revendications précédente, dans laquelle les polymères associatifs sont anioniques, cationiques, amphotères ou non ioniques.

14. Composition selon la revendication 13, dans laquelle au moins un des polymères associatifs est anionique.

15. Composition selon la revendication 14, dans laquelle les polymères associatifs anioniques comportent au moins un motif hydrophile de type acide carboxylique insaturé oléfinique et au moins un motif hydrophobe de type ester d'alkyl(C₁₀-C₃₀) d'acide carboxylique insaturé.

16. Composition selon la revendication 15, dans laquelle les polymères associatifs anioniques sont constitués d'acide acrylique, d'ester d'alkyl(C₁₂-C₂₂) d'acide acrylique ou métacrylique et d'agent réticulant.

17. Composition selon la revendication 16, dans laquelle les polymères associatifs anioniques sont constitués de 95 à 60 % en poids d'acide acrylique, de 4 à 40 % en poids d'acrylate d'alkyle en C₁₀-C₃₀ et de 0 à 6 % en poids de monomère insaturé polyéthylénique copolymérisable.

18. Composition selon la revendication 16, dans laquelle les polymères associatifs anioniques sont constitués de 98 à 96 % en poids d'acide acrylique, de 1 à 4 % en poids d'acrylate d'alkyle en C₁₀-C₃₀ et de 0,1 à 0,6 % en poids de monomère polymérisable réticulant.

19. Composition selon la revendication 14, dans laquelle les polymères associatifs anioniques sont des polyuréthanes.

20. Composition selon la revendication 13, dans laquelle au moins un des polymères associatifs est cationique.

21. Composition selon la revendication 20, dans laquelle les polymères associatifs cationiques sont des dérivés de cellulose quatemisée.

22. Composition selon la revendication 21, dans laquelle les dérivés de cellulose quatemisée sont des hydroxyéthylcelluloses quaternisées modifiées par des groupements comportant au moins une chaîne grasse.

23. Composition selon la revendication 22, dans laquelle la chaîne grasse des hydroxyéthylcelluloses est un radical alkyle comportant de 8 à 30 atomes de carbone.

24. Composition selon la revendication 20, dans laquelle les polymères associatifs cationiques sont des polyuréthanes.

25. Composition selon la revendication 13, dans laquelle au moins un des polymères associatifs est non ionique et choisi parmi les polyéthers polyuréthanes.

26. Composition selon la revendication 25, dans laquelle les polyéthers polyuréthanes sont des polycondensats de polyéthylèneglycol à 150 ou 180 moles d'oxyde d'éthylène, d'alcool stéarylique et d'au moins un diisocyanate.

27. Composition selon la revendication 26, dans laquelle les polyéthers polyuréthanes sont des polycondensats de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81 %).

28. Composition selon la revendication 25, dans laquelle les polyéthers polyuréthanes sont des polycondensats de polyéthylèneglycol à 150 ou 180 moles d'oxyde d'éthylène, d'alcool décylique et d'au moins un diisocyanate.

29. Composition selon la revendication 28, dans laquelle les polyéthers polyuréthanes sont des polycondensats de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%).

30. Composition selon l'une quelconque des revendications précédentes, comprenant au moins un coupleur additionnel choisi parmi les méta-phénylènediamines, les méta-aminophénols autres que le 2-chloro 6-méthyl 3-amino phénol, les méta-diphénols, les coupleurs naphtaléniques, les coupleurs hétérocycliques ainsi que leurs sels d'addition.

31. Composition selon l'une quelconque des revendications précédentes, dans laquelle la quantité de chacune des bases d'oxydation est comprise entre 0,001 à 10 % en poids environ du poids total de la composition tinctoriale.

32. Composition selon l'une quelconque des revendications précédentes, dans laquelle la quantité de chacun des polymères associatifs est comprise entre 0,01 à 10% en poids environ du poids total de la composition tinctoriale.

33. Composition selon l'une quelconque des revendications précédentes, dans laquelle la quantité de chacun des coupleurs est comprise entre 0,001 et 10 % en poids environ du poids total de la composition tinctoriale.

34. Composition selon l'une quelconque des revendications précédentes, comprenant un agent oxydant.

35. Composition selon la revendication 34, dans laquelle l'agent oxydant est choisi parmi le peroxyde d'hydrogène, le peroxyde d'urée, les bromates de métaux alcalins, les persels, les peracides et les enzymes oxydases.

36. Procédé de teinture d'oxydation des fibres kératiniques, dans lequel une composition telle que définie à l'une quelconque des revendications 1 à 33 est appliquée sur les fibres kératiniques, et que la couleur est révélée à l'aide d'un agent oxydant.

37. Procédé selon la revendication 36, dans lequel l'agent oxydant est choisi parmi le peroxyde d'hydrogène, le peroxyde d'urée, les bromates de métaux alcalins, les persels, les peracides et les enzymes oxydases.

38. Procédé selon la revendication 36 ou 37, dans lequel l'agent oxydant est mélangé au moment de l'emploi à la composition telle que définie à l'une quelconque des revendications 1 à 33.

39. Procédé selon la revendication 36 ou 37, dans lequel une composition comprenant l'agent oxydant est appliquée sur les fibres kératiniques simultanément ou séquentiellement à la composition telle que définie à l'une quelconque des revendications 1 à 33.

40. Dispositif à plusieurs compartiments, dans lequel un premier compartiment contient une composition tinctoriale telle que définie à l'une quelconque des revendications 1 à 33 et un deuxième compartiment contient une composition comprenant un agent oxydant.

41. Utilisation de la composition tinctoriale telle que définie à l'une quelconque des revendications 1 à 35 pour la teinture d'oxydation des fibres kératiniques.

## Claims

1. Composition for the oxidation dyeing of keratin fibres, comprising, in a suitable dyeing medium:
- at least one coupler chosen from 2-chloro-6-methyl-3-aminophenol and the addition salts thereof;
- at least one oxidation base; and
- at least one thickening associative polymer comprising at least one C₈-C₃₀ fatty chain.

2. Composition according to Claim 1, in which the oxidation base(s) is (are) chosen from para-phenylenediamines, double bases, para-aminophenols, ortho-aminophenols and heterocyclic oxidation bases, and the addition salts thereof.

3. Composition according to Claim 2, in which the para-phenylenediamines are chosen from the compounds of formula (I) below, and the addition salts thereof: 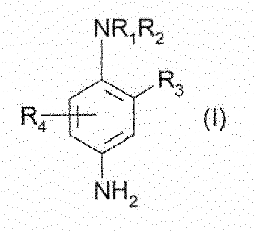 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄) alkyl radical, a C₁-C₄ alkyl radical substituted with a nitrogenous group, a phenyl radical or a 4'-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous group;
- R₁ and R₂ may form together, with the nitrogen atom to which they are attached, a 5- to 8-membered heterocycle optionally substituted with one or more groups chosen from C₁-C₄ alkyl, amino, C₁-C₄ hydroxyalkyl and trialkylammonium radicals;
- R₃ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)alkoxy radical, a mesylamino (C₁-C₄) alkoxy radical or a carbamoylamino(C₁-C₄)alkoxy radical;
- R₄ represents a hydrogen or halogen atom or a C₁-C₄ alkyl radical.

4. Composition according to Claim 3, in which the para-phenylenediamines of formula (I) are chosen from para-phenylenediamine, para-tolylenediamine, 2-chloro-para-phenylenediamine, 2,3-dimethyl-para-phenylene-diamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-2-methylaniline, 4-amino-N,N-bis(β-hydroxyethyl)-2-chloroaniline, 2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxy-ethyloxy)-para-phenylenediamine, 2-(β-acetylamino-ethyloxy)-para-phenylenediamine, N-(β-methoxyethyl)-para-phenylenediamine and the addition salts thereof.

5. Composition according to any one of Claims 2 to 4, in which the double bases are chosen from the compounds corresponding to formula (II) below, and the addition salts thereof: 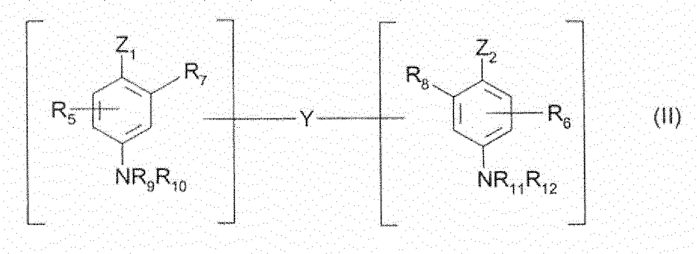 in which:
- Z₁ and Z₂, which may be identical or different, represent a hydroxyl or amino radical that may be substituted with a C₁-C₄ alkyl radical or with a linker arm Y;
- the linker arm Y represents a linear or branched alkylene chain containing from 1 to 14 carbon atoms, that may be interrupted by or terminated with one or more nitrogenous groups and/or one or more hetero atoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y, a C₁-C₄ alkyl radical or a C₁-C₄ monohydroxyalkyl radical;
it being understood that the compounds of formula (II) contain only one linker arm Y per molecule.

6. Composition according to Claim 5, in which the doubles bases of formula (II) are chosen from N,N'-bis (β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis (β-hydroxyethyl)-N,N'-bis(4-aminophenyl)tetramethylenediamine, N,N'-bis (4-methylaminophenyl)tetramethylenediamine, N,N'-bis-(ethyl)-N,N'-bis(4'-amino-3'-methylphenyl)ethylenedi-amine, 1,8-bis(2,5-diaminophenoxy)-3,5-dioxaoctane, 4-amino-6-[(5'-amino-2'-hydroxy-3'-methylphenyl)methyl]-2-methylphenol and bis(5-amino-2-hydroxyphenyl)methane, and the addition salts thereof.

7. Composition according to any one of Claims 2 to 6, in which the para-aminophenols are chosen from the compounds corresponding to formula (III) below, and the addition salts thereof: 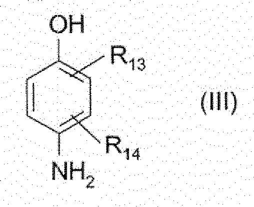 in which:
- R₁₃ represents a hydrogen atom, a halogen atom, or a C₁-C₄ alkyl, C₁-C₄ monohydroxyalkyl, (C₁-C₄) - alkoxy (C₁- C₄) alkyl, C₁-C₄ aminoalkyl or hydroxy(C₁-C₄)alkylamino(C₁-C₄)alkyl radical;
- R₁₄ represents a hydrogen atom, a halogen atom, or a C₁-C₄-alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy-(C₁-C₄)alkyl radical;
it being understood that at least one of the radical R₁₃ and R₁₄ represents a hydrogen atom.

8. Composition according to Claim 7, in which the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, 4-amino-2-chlorophenol and 4-amino-2,6-dichlorophenol, the addition salts thereof.

9. Composition according to any one of Claims 2 to 8, in which the ortho-aminophenols are chosen from 2-aminophenol, 2-amino-5-methylphenol, 2-amino,-6-methylphenol and 5-acetamido-2-aminophenol, and the addition salts thereof.

10. Composition according to any one of Claims 2 to 9, in which the heterocyclic bases are chosen from pyridine derivatives, pyrimidine derivatives, pyrazole derivatives and pyrazolopyrimidine derivatives, and the addition salts thereof.

11. Composition according to any one of the preceding claims, in which the associative polymers comprise hydrophilic zones and hydrophobic zones **characterized by** at least one C₈-C₃₀ fatty chain.

12. Composition according to any one of the preceding claims, in which the associative polymers are derived either from a free-radical polymerization or from a polycondensation starting with at least one monomer, at least one of which contains a C₈-C₃₀ fatty chain, or from the grafting onto a polymer of a compound comprising at least one C₈-C₃₀ fatty chain.

13. Composition according to any one of the preceding claims, in which the associative polymers are anionic, cationic, amphoteric or nonionic.

14. Composition according to Claim 13, in which at least one of the associative polymers is anionic.

15. Composition according to Claim 14, in which the anionic associative polymers comprise at least one hydrophilic unit of olefinic unsaturated carboxylic acid type and at least one hydrophobic unit of (C₁₀-C₃₀) alkyl ester of unsaturated carboxylic acid type.

16. Composition according to Claim 15, in which the anionic associative polymers consist of acrylic acid, an acrylic or methacrylic acid (C₁₂-C₂₂)alkyl ester and a crosslinking agent.

17. Composition according to Claim 16, in which the anionic associative polymers consist of from 95% to 60% by weight of acrylic acid, from 4% to 40% by weight of a C₁₀-C₃₀ alkyl acrylate and from 0 to 6% by weight of copolymerizable polyethylenic unsaturated Monomer.

18. Composition according to Claim 16, in which the anionic associative polymers consist of from 98% to 96% by weight of acrylic acid, from 1% to 4% by weight of a C₁₀-C₃₀ alkyl acrylate and from 0.1% two 0.6% by weight of crosslinking polymerizable monomer.

19. Compositions according to Claim 14, in which the anionic associative polymers are polyurethanes.

20. composition according to Claim 13, in which at least one of the associative polymers is cationic.

21. Composition according to Claim 20, in which the cationic associative polymers are quaternized cellulose derivatives.

22. Composition according to Claim 21, in which the quaternized cellulose derivatives are quaternized hydroxyethylcellulases modified with groups comprising at least one fatty chain.

23. Composition according to Claim 22, in which the fatty chain of the hydroxyethylcelluloses is an alkyl radical containing from 8 to 30 carbon atoms.

24. Composition according to Claim 20, in which the cationic associative polymers are polyurethanes.

25. Composition according to Claim 13, in which at least one of the associative polymers is nonionic and chosen from polyurethane polyethers.

26. Composition according to Claim 25, in which the polyurethane polyethers are polycondensates of polyethylene glycol containing 150 or 180 mol of ethylene oxide, of stearyl alcohol and of at least one diisocyanate.

27. Composition according to Claim 26, in which the polyurethane polyethers are polycondensates of polyethylene glycol containing 150 or 180 mol of ethylene oxide, of stearyl alcohol and of methylenebis(4-cyclohexyl isocyanate) (SMDI), at a concentration of 15% by weight in a matrix of maltodextrin (4%) and of water (81%).

28. Composition according to Claim 25, in which the polyurethane polyethers are polycondensats of polyethylene glycol containing 150 or 180 mol of ethylene oxide, of decyl alcohol and of at least one diisocyanate.

29. Composition according to Claim 28, in which the polyurethane polyethers are polycondensates of polyethylene glycol containing 150 or 180 mol of ethylene oxide, of decyl alcohol and of methylenebis(4-cyclohexyl isocyanate) (SMDI), at a concentration of 35% by weight in a mixture of propylene glycol (39%) and water (26%).

30. Composition according to any one of the preceding claims, comprising at least one additional coupler chosen from meta-phenylenediamines, meta-aminophenols other than 2-chloro-6-methyl-3-aminophenol, meta-diphenols, naphthalene-based couplers and heterocyclic couplers, and the addition salts thereof.

31. Composition according to any one of the preceding claim, in which the amount of each of the oxidation bases is between 0.001% and 10% by weight approximately relative to the total weight of the dye composition.

32. Composition according to any one of the preceding claims, in which the amount of each of the associative polymers is between 0.01% and 10% by weight approximately relative to the total weight of the dye composition.

33. Composition according to any one of the receding claims, in which the amount of each of the couplers is between 0.001% and 10% by weight approximately relative to the total weight of the dye composition.

34. Composition according to any one of the preceding claims, comprising an oxidizing agent.

35. Composition according to Claim 34, in which the oxidizing agent is chosen from hydrogen peroxide, urea peroxide, alkali metal bromates, persalts, peracids and oxidase enzymes.

36. Process for the oxidation dyeing of keratin fibres, in which a composition as defined in any one of Claims 1 to 33 is applied to the keratin fibres, and the colour is developed using an oxidizing agent.

37. Process according to Claim 36, in which the oxidizing agent is chosen from hydrogen peroxide, urea peroxide, alkali metal bromates, persalts, peracids and oxidase enzymes.

38. Process according to Claim 36 or 37, in which the oxidizing agent is mixed at the time of use with the composition as defined in any one of Claims 1 to 33.

39. Process according to Claim 36 or 37, in which a composition comprising the oxidizing agent is applied to keratin fibres simultaneously with or sequentially to the composition as defined in any one of Claims 1 to 33.

40. Multi-compartment device, in which a first compartment contains a dye composition as defined in any one of Claims 1 to 33 and a second compartment contains a composition comprising an oxidizing agent.

41. Use of the dye composition as defined in any one of Claims 1 to 35, for the oxidation dyeing of keratin fibres.

## Patentansprüche

1. Zusammensetzung zum Oxidationsfärben von Keratinfasern, umfassend in einem geeigneten Färbemedium:
- mindestens einen Kuppler, der unter 2-chlor-6-methyl-3-aminophenol und Additionssalzen davon ausgewählt ist;
- mindestens eine Oxidationsbase und
- mindestens ein Assoziativverdicker-Polymer mit mindestens einer C₈-C₃₀-Fettkette.

2. Zusammensetzung nach Anspruch 1, in der die Oxidationsbase bzw. die Oxidationsbasen unter para-Phenylendiaminen, Doppelbasen, para-Aminophenolen, ortho-Aminophenolen, heterocyclischen Oxidationsbasen und Additionssalzen davon ausgewählt sind.

3. Zusammensetzung nach Anspruch 2, in der die paraPhenylendiamine unter den Verbindungen der nachstehenden Formel (I) und Additionssalzen davon ausgewählt sind: 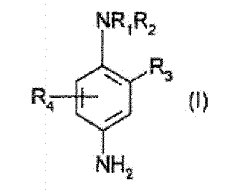 worin:
- R₁ für ein Wasserstoffatom, einen C₁-C₄-Alkylrest, einen C₁-C₄-Monohydroxyalkylrest, einen C₂-C₄-Polyhydroxyalkylrest, einen (C₁-C₄) -Alkoxy-(C₁-C₄)-alkylrest, einen C₁-C₄-Alkylrest, der durch eine stickstoffhaltige Gruppe substituiert ist, einen Phenylrest oder einen 4'-Aminophenylrest steht;
- R₂ für ein Wasserstoffatom, einen C₁-C₄-Alkylrest, einen C₁-C4-Monohydroxyalkylrest, einen C₂-C₄-Polyhydroxyalkylrest, einen (C₁-C₄)-Alkoxy-(C₁-C₄)-alkylrest oder einen C₁-C₄-Alkylrest, der durch eine stickstoffhaltige Gruppe substituiert ist, steht;
- R₁ und R₂ mit dem Stickstoffatom, an das sie gebunden sind, einen 5- bis 8-gliedrigen Heterocyclus, der gegebenenfalls durch eine oder mehrere unter C₁-C₄-Alkyl-, Amino-, Hydroxyalkyl- und Trialkylammoniumresten ausgewählte Gruppen substituiert ist, bilden können;
- R₃ für ein Wasserstoffatom, ein Halogenatom wie ein Chlor-, Brom-, Iod- oder Fluoratom, einen C₁-C₄-Alkylrest, einen C₂-C₄-Monohydroxyalkylrest, einen C₁-C₄-Hydroxyalkoxyrest, einen Acetylamino-C₁-C₄-alkoxyrest, einen Mesylamino-C₁-C₄-alkoxyrest oder einen Carbamoylamino-C₁-C₄-alkoxyrest steht;
- R₄ für ein Wasserstoffatom, ein Halogenatom oder einen C₁-C₄-Alkylrest steht.

4. Zusammensetzung nach Anspruch 3, in der die paraPhenylendiamine der Formel (I) unter para-Phenylendiamin, para-Tolylendiamin, 2-Chlor-para-phenylendiamin, 2,3-Dimethyl-para-phenylendiamin, 2,6-Dimethyl-para-phenylendiamin, 2,6-Diethyl-para-phenylendiamin, 2,5-Dimethyl-para-phenylen-diamin, N,N-Dimethyl-para-phenylendiamin, N,N-Diethyl-para-phenylendiamin, N,N-Dipropyl-para-phenylendiamin, 4-Amino-N,N-diethyl-3-methylanilin, N,N-Bis(β-hydroxyethyl)-para-phenylendiamin, 4-Amino-N,N-bis(β-hydroxyethyl)-2-methylanilin, 4-Amino-N,N-bis(β-hydroxyethyl)-2-chloranilin, 2-(β-Hydroxyethyl)-para-phenylendiamin, 2-Fluor-para-phenylendiamin, 2-Isopropyl-para-phenylendiamin, N-(β-hydroxypropyl)-para-phenylendiamin, 2-Hydroxymethyl-para-phenylendiamin, N,N-Dimethyl-3-methyl-para-phenylendiamin, N-Ethyl-N-(β-hydroxyethyl)-para-phenylendiamin, N-(β,γ-Dihydroxypropyl)-para-phenylendiamin, N-(4'-Aminophenyl)-para-phenylendiamin, N-Phenyl-para-phenylendiamin, 2-(β-Hydroxyethyloxy)-para-phenylendiamin, 2-(β-Acetylaminoethyloxy)-para-phenylendiamin, N-(β-Methoxyethyl)amino-para-phenylendiamin und Additionssalzen davon ausgewählt sind.

5. Zusammensetzung nach einem der Ansprüche 2 bis 4, in der die Doppelbasen unter den Verbindungen der nachstehenden Formel (II) und Additionssalzen davon ausgewählt sind: 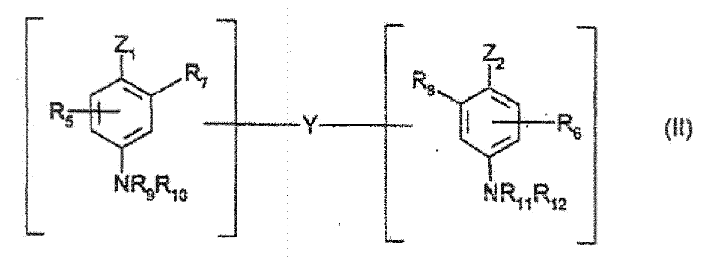 worin:
- Z₁ und Z₂ gleich oder verschieden sind und für einen Hydroxyl- oder Aminorest, der durch einen C₁-C₄-Alkylrest oder durch einen Linkerarm Y substituiert sein kann, stehen;
- der Linkerarm Y für eine lineare oder verzweigte Alkylenkette mit 1 bis 14 Kohlenstoffatomen, die durch eine oder mehrere stickstoffhaltige Gruppen und/oder durch ein oder mehrere Heteroatome wie Sauerstoff-, Schwefel- oder Stickstoffatome unterbrechen oder terminiert und gegebenenfalls durch einen oder mehrere Hydroxyd- oder C₁-C₆-Alkoxyreste substituiert sein kann, steht;
- R₅ und R₆ für ein Wasserstoffatom, ein Halogenatom, einen C₁-C₄-Alkylrest, einen C₁-C₄-Monohydroxyalkylrest, einen C₂-C₄-Polyhydroxy-alkylrest, einen C₁-C₄-Aminoalkylrest oder einen Linkerarm Y stehen;
- R₇, R₈, R₉, R₁₀, R₁₁ und R₁₂ gleich oder verschieden sind und für ein Wasserstoffatom, einen Linkerarm Y, einen C₁-C₄-Alkylrest oder einen C₁-C₄-Monohydroxyalkylrest stehen;
mit der Maßgabe, dass die Verbindungen der Formel (II) nur einen Linkerarm Y pro Molekül entfalten.

6. Zusammensetzung nach Anspruch 5, in der die Doppelbasen der Formel (II) unter N,N'-Bis(β-hydroxyethyl)-N,N'-bis(4'-aminophenyl)-1,3-diaminopropanol, N,N'-Bis(β-hydroxyethyl)-N,N'-bis-(4'-ammophenyl)ethylendiamin, N,N'-Bis(4-aminophenyl)tetramethylendiamin, N,N'-Bis(β-hydroxy-ethyl)-N,N'-bis(4-aminophenyl)tetramethylendiamin, N,N'-Bis(4-methylaminophenyl)tetramethylendiamin, N,N'-Bis(ethyl)-N,N'-bis(4'-amino-3'-methyl-phenyl)ethylendiamin, 1,8-Bis(2,5-diaminophenoxy)-3,5-dioxaoctan, 4-Amino-6-[(5'-amino-2'-hydroxy-3'-methylphenyl)methyl]-2-methylphenol, Bis(5-amino-2-hydroxyphenyl)methan und Additionssalzen davon ausgewählt sind.

7. Zusammensetzung nach einem der Anspruche 2 bis 6, in der die para-Aminophenole unter den Verbindungen der nachstehenden Formel (III) und Additionssalzen davon ausgewählt sind: 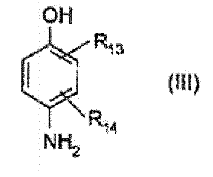 worin:
- R₁₃ für ein Wasserstoffatom, ein Halogenatom, einen C₁-C₄-Alkylrest, einen C₁-C₄-Monohydroxy-alkylrest, einen (C₁-C₄)-Alkoxy-(C₁-C₄)-alkyl-rest, einen C₁-C₄-Aminoalkylrest oder einen Hydroxy- (C₁-C₄) -alkylamino-C₁-C₄-alkylrest steht,
- R₁₄ für ein Wasserstoffatom, ein Halogenatom, einen C₁-C₄Alkylrest, einen C₁-C₄-Monohydroxy-alkylrest, einen C₂-C₄-Polyhydroxyalkylrest, einen C₁-C₄-Aminoalkylrest, einen C₁-C₄-Cyanoalkylrest oder einen (C₁-C₄)-Alkoxy-(C₁-C₄) - alkylrest steht,
mit der Maßgabe, dass mindestens einer der Reste R₁₃ und R₁₄ für ein Wasserstoffatom steht.

8. Zusammensetzung nach Anspruch 7, in der die para-Aminophenole unter para-Aminophenol, 4-amino-3-methylphenol, 4-Amino-3-fluorphenol, 4-Amino-3-hydroxymethylphenol, 4-Amino-2-methylphenol, 4-Amino-2-hydroxymethylphenol, 4-Amino-2-methoxy-methylphenol, 4-Amino-2-aminomethylphenol, 4-Amino-2-(β-hydroxyethylaminomethyl)phenol, 4-Amino-2-fluorphenol, 4-Amino-2-chlorphenol, 4-Amino-2,6-dichlorphenol und Additionssalzen davon ausgewählt sind.

9. Zusammensetzung nach einem der Ansprüche 2 bis 8, in der die ortho-Aminophenole unter 2-aminophenol, 2-Amino-5-methylphenol, 2-Amino-6-methylphenol, 5-Acetamido-2-aminophenol und Additionssalzen davon ausgewählt sind.

10. Zusammensetzung nach einem der Ansprüche 2 bis 9, in der die heterocyclischen Basen unter Pyridinderivaten, Pyrimidinderivaten, Pyrazolderivaten, Pyrazolopyrimidinderivaten und Additionssalzen davon ausgewählt sind.

11. Zusammensetzung nach einem der vorhergehenden Ansprüche, in der die Assoziativpolymere hydrophile Zonen und hydrophobe Zonen, die durch mindestens eine C₈-C₃₀-Fettkette **gekennzeichnet** sind, umfassen.

12. Zusammensetzung nach einem der vorhergehenden Ansprüche, in der die Assoziativpolymere entweder aus einer radikalischen Polymerisation oder einer Polykondensation ausgehend von mindestens einem Monomer, wovon mindestens eines eine C₈-C₃₀-Fettkette aufweist, oder aus der Pfropfung einer Verbidung mit mindestens einer C₈-C₃₀-Fettkette auf ein Polymer stammen.

13. Zusammensetzung nach einem der vorhergehenden Ansprüche, in der die Assoziativpolymere anionisch, kationisch, amphoter oder nichtionisch sind.

14. Zusammensetzung nach Anspruch 13, in der mindestens eines der Assoziativpolymere anionisch ist.

15. Zusammensetzung nach Anspruch 14, in der die anionischen Assoziativpolymere mindestens eine hydrophile Einheit vom Typ olefinisch ungesättigte carbonsaure und mindestens eine hydrophobe Einheit vom Typ (C₁₀-C₃₀)-Alkylester einer ungesättigten Carbonsäure umfassen.

16. Zusammensetzung nach Anspruch 15, in der die anionischen Assoziativpolymere aus Acrylsäure, Acrylsäure- oder Methacrylsäure-(C₁₂-C₂₂)-alkylester und Vernetzungsmittel bestehen.

17. Zusammensetzung nach Anspruch 16, in der die anionischen Assoziativpolymere aus 95 bis 60 Gew.-% Acrylsäure, 4 bis 40 Gew.-% C₁₀-C₃₀-Alkylacrylat und 0 bis 6 Gew.-% copolymerisierbarem polyethylenisch ungesättigtem Monomer bestehen.

18. Zusammensetzung nach Anspruch 16, in der die anionischen Assoziativpolymere aus 98 bis 96 Gew.-% Acrylsäure, 1 bis 4 Gew.-% C₁₀-C₃₀-Alkylacrylat und 0,1 bis 0,6 Gew.-% vernetzendem polymensierbarem Monomer bestehen.

19. Zusammensetzung nach Anspruch 14, in der es sich bei den anionischen Assoziativpolymeren um Polyurethane handelt.

20. Zusammensetzung nach Anspruch 13, in der mindestens eines der Assoziativpolymere kationisch ist.

21. Zusammensetzung nach Anspruch 20, in der es sich bei den kationischen Assoziativpolymeren um quaternisierte Cellulosederivate handelt.

22. Zusammensetzung nach Anspruch 21, in der es sich bei den quaternisierten Cellulosederivaten um quaternisierte Hydroxyethylcellulosen, die durch Gruppen mit mindestens einer Fettkette modifiziert sind, handelt.

23. Zusammensetzung nach Anspruch 22, in der es sich bei der Fettkette der Hydroxyethylcellulosen um einen Allylrest mit 8 bis 30 Kohlenstoffatomen handelt.

24. Zusammensetzung nach Anspruch 20, in der es sich bei den kationischen Assoziativpolymeren um Polyurethane handelt.

25. Zusammensetzung nach Anspruch 13, in der mindestens eines der Assoziativpolymere nichtionisch ist und unter Polyurethanpolyethern ausgewählt ist.

26. Zusammensetzung nach Anspruch 25, in der es sich bei den Polyurethanpolyethern um Polykondensate von Polyethylenglykol mit 150 oder 180 mol Ethylenoxid, Stearylalkohol und mindestens einem Diisocyanat handelt.

27. Zusammensetzung nach Anspruch 26, in der es sich bei den Polyurethanpolyethern um Polykondensate von Polyethylenglykol mit 150 oder 180 mol Ethylenoxid, Stearylalkohol und Methylenbis(4-cyclohexylisocyanat) (SMDI) in einer Konzentration von 15 Gew.-% in einer Matrix von Maltodextrin (4%) und Wasser (81%) handelt.

28. Zusammensetzung nach Anspruch 25, in der es sich bei den Polyurethanpolyethern um Polykondensate von Polyethylenglykol mit 150 oder 180 mol Ethylenoxid, Decylalkohol und mindestens einem Diisocyanat handelt.

29. Zusammensetzung nach Anspruch 28, in der es sich bei den Polyurethanpolyethern um Polykondensate von Polyethylenglykol mit 150 oder 180 mol Ethylenoxid, Decylalkohol und Methylenbis(4-cyclohexylisocyanat) (SMDI) in einer Konzentration von 35 Gew.-% in einer Mischung von Propylenglykol (39%) und Wasser (26%) handelt.

30. Zusammensetzung nach einem der vorhergehenden Ansprüche, umfassend mindestens einen zusätzlichen Kuppler, der unter meta-Phenylendiaminen, anderen meta-Aminophenolen als 2-Chlor-6-methyl-3-aminophenol, meta-Diphenolen, Kupplern auf Naphthalinbasis, heterocyclischen Kupplern sowie Additionssalzen davon ausgewählt ist.

31. Zusammensetzung nach einem der vorhergehenden Ansprüche, in der die Menge jeder der Oxidationsbasen zwischen ungefähr 0,001 und 10 Gew.-%, bezogen auf das Gesamtgewicht der Färbezusammensetzung, liegt.

32. Zusammensetzung nach einem der vorhergehenden Ansprüche, in der die Menge jedes der Assoziativpolymere zwischen ungefähr 0,01 und 10 Gew.-%, bezogen auf das Gesamtgewicht der Färbezusammensetzung, liegt.

33. Zusammensetzung nach einem der vorhergehenden Ansprüche, in der die Menge jedes der Kuppler zwischen ungefähr 0,001 und 10 Gew.-%, bezogen auf Gesamtgewicht der Färbezusammensetzung, liegt.

34. Zusammensetzung nach einem der vorhergehenden Ansprüche, umfassend ein Oxidationsmittel.

35. Zusammensetzung nach Anspruch 34, in der das Oxidationsmittel unter Wasserstoffperoxid, Harnstoffperoxid, Alkalimetallbromaten, Persalzen, Persäuren und Oxidaseenzymen ausgewählt ist.

36. Verfahren zum Oxidationsfärben von Keratinfasern, bei dem man auf die Keratinfasern eine Zusammensetzung gemäß einem der Ansprüche 1 bis 33 aufbringt und die Farbe mit Hilfe eines Oxidationsmittels entwickelt.

37. Verfahren nach Anspruch 36, bei dem man das Oxidationsmittel unter Wasserstoffperoxid, Harnstoffperoxid, Alkalimetallbromaten, Persalzen, Persäuren und Oxidaseenzymen auswählt.

38. Verfahren nach Anspruch 36 oder 37, bei dem man das Oxidationsmittel zum Zeitpunkt der Verwendung mit der Zusammensetzung gemäß einem der Ansprüche 1 bis 33 mischt.

39. Verfahren nach Anspruch 36 oder 37, bei dem man auf die Keratinfasern gleichzeitig mit oder nach der Zusammensetzung gemäß einem der Ansprüche 1 bis 33 eine das Oxidationsmittel umfassende Zusammensetzung aufbringt.

40. Vorrichtung mit mehreren Kompartimenten, in der ein erstes Kompartiment eine Färbezusammensetzung gemäß einem der Ansprüche 1 bis 33 enthält und ein zweites Kompartiment eine ein Oxidationsmittel umfassende Zusammensetzung enthält.

41. Verwendung der Färbezusammensetzung gemäß einem der Ansprüche 1 bis 35 zum oxidationsfärben von Keratinfasern.
